Europäisches Patentamt

European Patent Office

Office européen des brevets

(11) Veröffentlichungsnummer: **0 205 922 B1**

(12) **EUROPÄISCHE PATENTSCHRIFT**

(45) Veröffentlichungstag der Patentschrift: **19.08.92**

(21) Anmeldenummer: **86106830.2**

(22) Anmeldetag: **20.05.86**

Die Akte enthält technische Angaben, die nach dem Eingang der Anmeldung eingereicht wurden und die nicht in dieser Patentschrift enthalten sind.

(51) Int. Cl.⁵: **A61K 7/08**, A61K 7/50

(54) **Fliessfähiges Perlglanzkonzentrat.**

(30) Priorität: **28.05.85 DE 3519080**

(43) Veröffentlichungstag der Anmeldung:
**30.12.86 Patentblatt 86/52**

(45) Bekanntmachung des Hinweises auf die Patenterteilung:
**19.08.92 Patentblatt 92/34**

(84) Benannte Vertragsstaaten:
**AT BE CH DE FR GB IT LI NL SE**

(56) Entgegenhaltungen:
**EP-A- 0 108 517     EP-A- 0 158 174**
**DE-A- 3 212 420     FR-A- 1 574 317**
**GB-A- 1 540 386     GB-A- 2 121 072**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Scheuffgen, Ingeborg**
**Spulgasse 3**
**W-4040 Neuss(DE)**

**Beschreibung**

Gegenstand der Erfindung ist ein Perlglanzkonzentrat in Form einer fließfähigen, von ionogenen Tensiden freien, wäßrigen Dispersion.

Wäßrigen Zubereitungen von Tensiden und kosmetischen Präparaten kann man durch Einarbeitung von Substanzen, die nach dem Abkühlen in Form feiner, perlmuttartig aussehender Kristalle ausfallen und in den Zubereitungen dispergiert bleiben, ein perlglänzendes, ästhetisch ansprechendes Aussehen verleihen. Als perlglanzbildende Stoffe eignen sich z. B. die Mono- und Diester aus Ethylenglycol, Propylenglycol und oligomeren Alkylenglycolen dieser Art oder Glycerin mit $C_{16}$-$C_{22}$-Fettsäuren sowie Monoalkanolamide von $C_{12}$-$C_{22}$-Fettsäuren mit Alkanolaminen mit 2 oder 3 C-Atomen.

Es ist auch bekannt, die genannten Perlglanzbildner in Wasser oder in wäßrigen Tensidlösungen stabil zu dispergieren und die auf diese Weise erhaltenen konzentrierten Perlglanzdispersionen den perlglänzend auszustattenden Zubereitungen ohne Erwärmung zuzusetzen, so daß sich das für die Einarbeitung sonst erforderliche Erwärmen und Abkühlen zur Bildung der Perlglanzkristalle erübrigt.

Perlglanzkonzentrate auf Basis der genannten Perlglanzbildner sind z. B. aus DE-A-16 69 152 und aus JP-56/71021 (Chem. Abstr. 95/156360) bekannt. Die aus JP-56/71021 bekannten Perlglanzkonzentrate haben den Nachteil daß sie nicht fließfähig sind und durch entsprechende Verdünnung mit Wasser keine stabilen fließfähigen Dispersionen ergeben. Dadurch wird die Handhabung und technische Verarbeitung der Konzentrate erheblich erschwert. Die aus DE-A-16 69 152 bekannten Perlglanzkonzentrate enthalten zur Stabilisierung der Disperion im flüssigen Zustand anionische Tenside. Gleichfalls zwingend anionische Tenside enthalten die perlglänzenden Antischuppen-Shampoos gemäß EP-A-108 517. Ein Gehalt an ionogenen Tensiden ist aber bei vielen Anwendungen solcher Perlglanzkonzentrate unerwünscht, da sich mit Rezepturbestandteilen entgegengesetzter Ionogenität Unverträglichkeiten und damit Störungen der Dispersionsstabilität ergeben können.

Schließlich werden in der DE-A-32 12 420 Dispersionen mit hohem Anteil an Perlglanzmitteln offenbart, bei denen das Auftreten flüssigkristalliner Phasen ausgenutzt wird. Diese Dispersionen benötigen aber einen für das Auftreten der Flüssigkristallphase notwendigen hohen Tensidanteil.

Es bestand daher weiterhin die Aufgabe, Perlglanzkonzentrate aufzufinden, die einerseits bei Raumtemperatur von ca. 10 - 20 °C noch fließfähig sind, keine ionogenen Tenside oder Dispergiermittel enthalten und trotzdem über viele Monate lagerstabil bleiben, ohne daß die Perlglanzkristalle sedimentieren oder sich an der Oberfläche absetzen.

Darüber hinaus sollen die Perlglanzkonzentrate einen bis mindestens 50 °C stabilen Perlglanz aufweisen, der auch in den damit ausgestatteten Zubereitungen bei schwankenden Temperaturen erhalten bleibt. Die Perlglanzkristalle sollen eine hohe Brillanz aufweisen und auch nach dem Aufschmelzen bzw. Lösen durch Erwärmung über den Schmelzpunkt sich beim Abkühlen in derselben brillanten, einheitlichen Kristallform wieder ausbilden.

Die vorgenannten Anforderungen werden erfüllt durch ein Perlglanzkonzentrat in Form einer fließfähigen, von ionogenen Tensiden freien Dispersion, dadurch gekennzeichnet, daß es

(A) 5 - 15 Gew.-% eines oder mehrerer Ester der allgemeinen Formel $R^1$ - $(OC_nH_{2n})_x$ - $OR^2$, in der $R^1$ eine lineare Fettacylgruppe mit 16 - 22 C-Atomen, $R^2$ Wasserstoff oder eine Acylgruppe $R^1$, n = 2 oder 3 und x eine Zahl von 1 - 4 ist,

(B) 1 - 6 Gew.-% eines oder mehrerer Monoethanolamide von Fettsäuren mit 12 - 22 C-Atomen,

(C) 1 - 5 Gew.-% eines oder mehrerer nichtionogener Anlagerungsprodukte von Ethylenoxid an Fettalkohole, Fettsäuren und Fettsäureamide oder Alkanolamide, wobei der Fettalkyl- oder Fettacylrest 12 bis 22 C-Atome trägt, an Alkylphenole mit 8 bis 16 C-Atomen in der Alkylgruppe und an Fettsäure-Polyol-Partialester, ausgewählt aus der Gruppe der Glycerinmonoester, Pentaerythritmonoester sowie Sorbitanmono- und -diester von Fettsäuren mit 12 bis 22 C-Atomen, mit einem HLB-Wert von 12 bis 16 enthält. Daneben enthalten die erfindungsgemäßen Perlglanzkonzentrate im wesentlichen Wasser in einer Menge von ca. 75 - 90 Gew.-%.

Als Ester der allgemeinen Formel $R^1(OC_nH_{2n})_x OR^2$ können z. B. die Mono- und Diester des Ethylenglycols und Propylenglycols mit höheren Fettsäuren, z. B. mit Palmitinsäure, Stearinsäure oder Behensäure oder die Diester des Diethylenglycols oder des Triethylenglycols mit solchen Fettsäuren eingesetzt werden. Geeignet sind auch Mischungen von Mono- und Diestern der genannten Glycole mit Fettsäuregemischen, z. B. mit gehärteter Talgfettsäure oder mit der gesättigten $C_{16}$-$C_{18}$-Fettsäurefraktion der Talgfettsäure. Bevorzugt geeignet ist der Ethylenglycolmono-und/oder Diester der Palmitin- und/oder Stearinsäure.

Als Monoethanolamide von Fettsäuren mit 12 - 18 C-Atomen können z. B. Laurinsäuremonoethanolamid, Myristinsäuremonoethanolamid, Palmitin-/Stearinsäure-Monoethanolamid und bevorzugt das Monoethanolamid der $C_{12}$-$C_{18}$-Fraktion der Kokosfettsäure verwendet werden.

2

Als nichtionogene Ethylenoxidaddukte eignen sich die Anlagerungsprodukte von Ethylenoxid an Fettalkohole, Fettsäuren und Fettsäureamide oder Alkanolamide, wobei der Fettalkyl- oder Fettacylrest 12 - 22 C-Atome trägt. Weiterhin eignen sich Ethylenoxidaddukte an Alkylphenole mit einer Alkylgruppe mit 8 - 16 C-Atomen. Schließlich eignen sich auch Ethylenoxidaddukte an Fettsäure-Polyol-Partialester, ausgewählt aus der Gruppe der Glycerinmonoester, der Pentaerythritmonoester, Sorbitanmono- und -diester von Fettsäuren mit 12 - 22 C-Atomen. Das Gewichtsverhältnis von hydrophilen zu lipophilen Gruppen in diesen Ethylenoxidaddukten sollte so sein, daß das Gewicht der hydrophilen Gruppen, also das Gewicht der (vom Ethylenoxid gebildeten) Polyethylenglykolethergruppen und der (bei Fettsäure-Polyol-Partialester-Addukten) Polyolgruppen etwa 60 - 80 Gew.-% des Gesamtmoleküls der Ethylenoxidaddukte ausmacht. Bei diesen für die Herstellung erfindungsgemäßer Perlglanzkonzentrate geeigneten Ethylenoxidaddukten liegt der HLB-Wert gemäß HLB = (E + P)/5 (wobei E = Gehalt Ethylenoxid in Gew.-% und P = Gehalt an mehrwertigem Alkohol in Gew.-% im Addukt), also im Bereich von 12 - 16.

Besonders gut geeignete nichtionogene Ethylenoxidaddukte sind die Anlagerungsprodukte von 6 - 20 Mol Ethylenoxid an Fettalkohole mit 12 - 22 C-Atomen.

Die erfindungsgemäßen Perlglanzkonzentrate weisen einen besonders seidig bis metallisch glänzenden Perlglanz auf, wenn als Perlglanzbildner eine Kombination aus

(A1) 5 - 8 Gew.-% eines Gemisches aus Ethylenglycolmono-und distearat, bevorzugt im Gewichtsverhältnis 1 : 2 bis 1 : 5

(A2) 2 - 5 Gew.-% Triethylenglycoldistearat

enthalten ist.

Eine besonders hohe Stabilität der Perlglanzkonzentrate wird dadurch erzielt, daß als Komponente C eine Kombination aus

(C1) 1 - 3 Gew.-% eines Anlagerungsproduktes von 8 - 15 Mol Ethylenoxid an einen $C_{12}$-$C_{18}$-Kokosfettalkoholschnitt und

(C2) 0,5 - 2 Gew.-% eines Anlagerungsproduktes von 10 - 20 Mol Ethylenoxid an einen gesättigten $C_{16}$-$C_{18}$-Fettalkoholschnitt

enthalten ist.

Geeignete $C_{12}$-$C_{18}$-Kokosfettalkoholschnitte werden aus Kokosöl durch katalytische Hydrierung der ungesättigten Anteile, Umesterung zum gesättigten Kokosfettsäuremethylester, katalytische Hydrierung zum gesättigten Kokosfettalkohol und destillative Abtrennung der $C_6$-, $C_8$- und $C_{10}$-Fettalkohole (sogenannte Kokosvorlauffettalkohole) gewonnen. Geeignete $C_{16}$-$C_{18}$-Fettalkoholschnitte lassen sich aus vielen pflanzlichen und tierischen Fetten und Ölen auf analoge Weise gewinnen. Die Anlagerung von Ethylenoxid an solche Fettalkoholschnitte erfolgt nach literaturbekannten Verfahren, z. B. in Gegenwart von basischen Katalysatoren wie z. B.. Kaliumhydroxid, Natriumhydoxid, Calciumacetat im Druckgefäß bei Temperaturen von 140 - 200 ° C.

Die erfindungsgemäßen Perlglanzkonzentrate sind bei Raumtemperatur, d. h. bei Temperaturen von 20 - 30 ° C fließfähig, d. h. sie lassen sich ohne zusätzliche Erwärmung aus Gefäßen ausgießen oder durch Rohre pumpen. Eine besonders gute Fließfähigkeit bei noch ausreichender Dispersionsstabilität weisen die erfindungsgemäßen Perlglanzkonzentrate auf, wenn die Komponenten A und B zusammen 8 - 14 Gew.-% des Konzentrates ausmachen. Ein ab ca. +5 ° C schon pumpfähiges, erfindungsgemäßes Perlglanzkonzentrat enthält

(A1) ca. 6 Gew.-% eines Gemisches aus Ethylenglycolmono-und -distearat im Gewichtsverhältnis 1 : 2 bis 1 : 5

(A2) ca. 4 Gew.-% Triethylenglycoldistearat

(B) 2 - 4 Gew.-% eines $C_{12}$-$C_{18}$-Kokosfettsäuremonoethanolamids

(C1) ca. 2 Gew.-% eines Anlagerungsproduktes von 8 - 12 Mol Ethylenoxid an einen gesättigten $C_{12}$-$C_{18}$-Kokosfettalkoholschnitt

(C2) ca. 1 Gew.-% eines Anlagerungsproduktes von 10 - 16 Mol Ethylenoxid an einen gesättigten $C_{16}$-$C_{18}$-Fettalkoholschnitt.

Anstelle des Ethylenglycolmono- und -distearats und des Triethylenglycoldistearats können auch die entsprechenden Ester von Palmitinsäure-Stearinsäure-Gemischen eingesetzt werden, wobei wenigstens 50 Gew.-% Stearinsäure im Gemisch vorliegen soll.

Neben den genannten obligatorischen Komponenten enthalten die erfindungsgemäßen Perlglanzkonzentrate im wesentlichen Wasser. In untergeordneten Mengen sind Konservierungsmittel, z. B. Formaldehyd, Na-Benzoat, Sorbinsäure, p-Hydroxybenzoesäureester, 5-Brom-5-nitro-1,3-dioxan oder andere für wäßrige Zubereitungen geeignete Konservierungsmittel enthalten. Weiterhin können in untergeordneten Mengen Puffersubstanzen zur Einstellung des pH-Wertes auf Werte zwischen 6 und 8, z. B. Citronensäure und/oder Natriumcitrat enthalten sein.

EP 0 205 922 B1

Die Herstellung der erfindungsgemäßen Perlglanzkonzentrate erfolgt bevorzugt in der Weise, daß die Komponenten A, B und C zunächst gemeinsam über ihren Schmelzpunkt, bevorzugt auf eine Temperatur von 75 - 100 °C erwärmt und vermischt werden. Zu dieser Schmelze wird dann das auf ebenfalls 75 - 100 °C erwärmte Wasser unter Rühren zugegeben. Das Wasser kann bereits Konservierungsmittel und Puffersubstanzen enthalten. Die entstehende Emulsion wird dann innerhalb von 5 - 20 Minuten auf ca. +50 °C unter Rühren gekühlt und bei dieser Temperatur mit einem Homogenisator oder Dispergieraggregat, welches hohe Scherkräfte entwickelt, für kurze Zeit, z. B. von 1 - 3 Minuten, homogenisiert. Hierfür eignen sich statische und dynamische Mischaggregate, z. B. Spalthomogenisatoren oder Dispergiergeräte, die nach dem Stator-Rotor-Prinzip arbeiten. Nach dieser kurzen und intensiven Homogenisierung wird die gebildete Dispersion unter langsamen Rühren weiter auf Raumtemperatur abgekühlt.

Die erfindungsgemäßen Perlglanzkonzentrate eignen sich zur Erzeugung von Perlglanz in wäßrigen Tensidzubereitungen beliebiger Ionogenität und in wäßrigen kosmetischen Zubereitungen gleichgültig ob diese kationische oder anionische Tenside oder Polymere enthalten. Zur Erzeugung von Perlglanz werden 1 - 10 Gew.-% der erfindungsgemäßen Perlglanzkonzentrate mit der wäßrigen Zubereitung verteilt. Die Verteilung der Perlglanzkonzentrate ist unter leichtem Rühren ohne Erwärmung, also bei Raumtemperaturen von 10 - 30 °C, möglich.

Die folgenden Beispiele sollen den Erfindungsgegenstand näher erläutern.

**Beispiele**

1. Herstellung eines fließfähigen Perlglanzkonzentrates Rezeptur:

| | |
|---|---|
| Ethylenglycolstearat (CTFA-Bezeichnung: Glycol Distearate) | 6,0 g |
| Triethylenglycoldistearat (Hydroxylzahl 13,5) (CTFA-Bezeichnung: PEG-3-Distearate) | 4,0 g |
| $C_{12}$-$C_{18}$-Kokosfettsäuremonoethanolamid | 3,5 g |
| $C_{12}$-$C_{18}$-Kokosfettalkohol + 10 Mol EO | 2,0 g |
| $C_{16}$-$C_{18}$-Fettalkohol (1:1) + 12 Mol EO | 1,0 g |
| Formaldehydlösung (10%ig) | 0,15 g |
| Wasser | ad 100 g |
| Zitronensäurelösung (1%ig) bis pH = 6,5 - 7,5 | |

Herstellverfahren:

Die Fettkomponenten wurden gemeinsam über ihren Schmelzpunkt erhitzt und bei 85 °C vermischt. Formaldehydlösung, Wasser und Zitronensäurelösung wurden ebenfalls gemischt, auf 85 °C erhitzt und unter Rühren zur Schmelze der Fettkomponenten gegeben. Nach 5 - 10 Minuten Rühren wurde die Dispersion innerhalb von 10 Minuten auf 50 °C unter weiterem Rühren abgekühlt, dann bei 50 °C mit einem IKA-ULtra-Turrax T 45-Dispergator 2 Minuten lang homogenisiert. Danach wurde unter langsamem Rühren auf Raumtemperatur (ca. 25 °C) abgekühlt.

Es wurde eine weiß-perlglänzende flüssige Dispersion mit folgenden Kennzahlen erhalten
Feststoffgehalt: 16,5 Gew.-%
pH-Wert 1%ige Lösung: 6,9
Viskosität (+20 °C): 3 500 mPa•s (Höppler-Kugelkel-Viskosimeter)
Viskosität (+5 °C): 1 300 mPa•s (Höppler-Kugelkel-Viskosimeter)

2. Anwendungsbeispiele

2.1 Ampholytisches Haarwaschmittel

4

| | |
|---|---|
| N-Kokosacylamidopropyl-dimethylglycin (30%ig) (CTFA-Bezeichnung: Cocoamidopropyl Betaine) | 30,0 g |
| Polyol-Fettsäureester (CETIOL (R) HE) (CTFA-Bezeichnung: PEG-7-Glyceryl-Cocoate) | 2,0 g |
| Kokosfettsäurediethanolamid (CTFA-Bezeichnung: Cocamide DEA) | 4,0 g |
| Perlglanzkonzentrat nach Beispiel 1 | 5,0 g |
| 5-Brom-5-nitro-1,3-dioxan, 10%ige Lösung in 1,2-Propylenglycol | 0,2 g |
| Natriumchlorid | 1,0 g |
| Zitronensäure-Lösung, 10%ig in Wasser | 0,5 g |
| Wasser | ad 100 g |

2.2 Anionisches Shampoo

| | |
|---|---|
| $C_{12}$-$C_{14}$-Fettalkoholpolyglycolethersulfat | 38,0 g |
| N-Kokosacylamidoethyl-hydroxyethylcarboxymethyl-glycin (CTFA-Bezeichnung: Cocoemphocarboxyglycinat) | 10,0 g |
| Polyol-Fettsäureester (Cetiol (R) HE) (CTFA-Bezeichnung: PEG-7-Glycerol-Cocoate) | 2,0 g |
| Kokosfettsäurediethanolamid | 2,0 g |
| Natriumchlorid | 1,5 g |
| Perlglanzkonzentrat nach Beispiel 1 | 5,0 g |
| 5-Brom-5-nitro-1,3-dioxan, 10%ige Lösung in 1,2-Propylenglycol | 0,2 g |
| Zitronensäurelösung, 10%ig in Wasser | 0,1 g |
| Wasser | ad 100 g |

2.3 Schnellhaarkur

| | |
|---|---|
| Tris-(oligooxyethyl)-alkylammoniumphosphat (CTFA-Bezeichnung: Quaternium-52) | 2,0 g |
| Polyol-Fettsäureester (Cetiol (R) HE) (CTFA-Bezeichnung: PEG-7-Glyceryl-Cocoate) | 0,5 g |
| Hydroxyethylcellulose (Viscontran (R) HEC 30 000 PR) 2%ig in Wasser | 50,0 g |
| Perlglanzkonzentrat nach Beispiel 1 | 2,0 g |
| Zitronensäure | 0,2 g |
| Wasser | 45,0 g |

2.4 Schnellhaarkur

| | |
|---|---|
| Polyglycol-Polyamin-Kondensationsharz (Polyquart (R) H 81) (CTFA-Bezeichnung: PEG-15-Tallow Polyamine) | 5,0 g |
| Hydroxyethylcellulose (Viscontran HEC 30 000 PR) 2%ig in Wasser | 93,0 g |
| Perlglanzkonzentrat nach Beispiel 1 | 2,0 g |

**Patentansprüche**

1. Perlglanzkonzentrat in Form einer fließfähigen, von ionogenen Tensiden freien Dispersion, dadurch gekennzeichnet, daß es

(A) 5 - 15 Gew.-% eines oder mehrerer Ester der allgemeinen Formel $R^1$-$(OC_nH_{2n})_xOR^2$, in der $R^1$ eine lineare Fettacylgruppe mit 16 - 22 C-Atomen, $R^2$ Wasserstoff oder eine Acylgruppe $R^1$, n = 2 oder 3 und x eine Zahl von 1 - 4 ist,

(B) 1 - 6 Gew.-% eines oder mehrerer Monoethanolamide von Fettsäuren mit 12 - 22 C-Atomen,

(C) 1 - 5 Gew.-% eines oder mehrerer nichtionogener Anlagerungsprodukte von Ethylenoxid an Fettalkohole, Fettsäuren und Fettsäureamide oder Alkanolamide, wobei der Fettalkyl- oder Fettacylrest 12 bis 22 C-Atome trägt, an Alkylphenole mit 8 bis 16 C-Atomen in der Alkylgruppe und an Fettsäure-Polyol-Partialester, ausgewählt aus der Gruppe der Glycerinmonoester, Pentaerythritmonoester sowie Sorbitanmono- und -diester von Fettsäuren mit 12 bis 22 C-Atomen, mit einem HLB-Wert von 12 bis 16

enthält.

2. Perlglanzkonzentrat nach Anspruch 1, dadurch gekennzeichnet, daß es

(A1) 5 - 8 Gew.-% eines Gemisches aus Ethylenglycolmono-und distearat und

(A2) 2 - 5 Gew.-% Triethylenglycoldistearat

enthält.

3. Fließfähiges Perlglanzkonzentrat nach den Ansprüchen 1 oder 2, dadurch gekennzeichnet, daß es

(C1)1 - 3 Gew.-% eines Anlagerungsproduktes von 8 - 15 Mol Ethylenoxid an einen $C_{12}$-$C_{18}$-Kokosfettalkoholschnitt und

(C2) 0,5 - 2 Gew.-% eines Anlagerungsproduktes von 10 - 20 Mol Ethylenoxid an einen gesättigten $C_{16}$-$C_{18}$-Fettalkoholschnitt

enthält.

4. Perlglanzkonzentrat nach den Ansprüchen 1 - 3, dadurch gekennzeichnet, daß die Komponenten A und B zusammen 8 - 14 Gew.-% des Konzentrates ausmachen.

5. Perlglanzkonzentrat nach den Ansprüchen 1 - 4, dadurch gekennzeichnet, daß es

(A1) ca. 6 Gew.-% eines Gemisches aus Ethylenglycolmono-und -distearat im Gewichtsverhältnis 1 : 2 bis 1 : 5,

(A2) ca. 4 Gew.-% Triethylenglycoldistearat,

(B) 2 - 4 Gew.-% eines $C_{12}$-$C_{18}$-Kokosfettsäuremonoethanolamids,

(C1) ca. 2 Gew.-% eines Anlagerungsproduktes von 8 - 12 Mol Ethylenoxid an einen $C_{12}$-$C_{18}$-Kokosfettalkoholschnitt,

(C2) ca. 1 Gew.-% eines Anlagerungsproduktes von 10 - 16 Mol Ethylenoxid an einen gesättigten $C_{16}$-$C_{18}$-Fettalkoholschnitt

enthält.

## Claims

1. A pearlescent concentrate in the form of a free-flowing dispersion free from ionic surfactants, characterized in that it contains

(A) 5 to 15° by weight of one or more esters corresponding to the general formula $R^1$ - $(OC_nH_{2n})_x$ - $OR^2$, in which $R^1$ is a linear $C_{16-22}$ fatty acyl group, $R^2$ is hydrogen or an acyl group $R^1$, n = 2 or 3 and x is a number of 1 to 4,

(B) 1 to 6% by weight of one or more monoethanolamides of $C_{12-22}$ fatty acids,

(C) 1 to 5% by weight of one or more nonionic adducts of ethylene oxide with fatty alcohols, fatty acids and fatty acid amides or alkanolamides, the fatty alkyl or fatty acyl group containing 12 to 22 C atoms, with alkylphenols containing 8 to 16 C atoms in the alkyl group and with fatty acid/polyol partial esters selected from the group consisting of glycerol monoesters, pentaerythritol monoesters and sorbitan mono-and diesters of $C_{12-22}$ fatty acids having an HLB value of 12 to 16.

2. A pearlescent concentrate as claimed in claim 1, characterized in that it contains

(A1) 5 to 8% by weight of a mixture of ethylene glycol mono- and distearate and

(A2) 2 to 5% by weight of triethylene glycol distearate.

3. A free-flowing pearlscent concentrate as claimed in claim 1 or 2, characterized in that it contains
(C1) 1 to 3% by weight of an adduct of 8 to 15 mol of ethylene oxide with a $C_{12-18}$ coconut oil fatty alcohol cut and
(C2) 0.5 to 2% by weight of an adduct of 10 to 20 mol of ethylene oxide with a saturated $C_{16-18}$ fatty alcohol cut.

4. A pearlescent concentrate as claimed in claims 1 to 3, characterized in that components A and B together make up from 8 to 14% by weight of the concentrate.

5. A pearlescent concentrate as claimed in claims 1 to 4, characterized in that it contains
(A1) approx. 6% by weight of a mixture of ethylene glycol mono- and distearate in a ratio by weight of 1:2 to 1:5,
(A2) approx. 4% by weight of triethylene glycol distearate,
(B) 2 to 4% by weight of a $C_{12-18}$ coconut oil fatty acid monoethanolamide;
(C1) approx. 2% by weight of an adduct of 8 to 12 mol ethylene oxide with a $C_{12-18}$ coconut oil fatty alcohol cut,
(C2) approx. 1% by weight of an adduct of 10 to 16 mol ethylene oxide with a saturated $C_{16-18}$ fatty alcohol cut.

## Revendications

1. Concentrat nacré sous forme d'une dispersion dépourvue d'agents tensio-actifs ionogènes, s'écoulant librement, caractérisé en ce qu'il contient:
A) de 5 à 15 % en poids d'un ou plusieurs esters de formule générale :

$$R^1 (OC_nH_{2n})_x OR_2$$

dans laquelle R₁ est un groupe acyle gras linéaire ayant de 16 à 22 atomes de carbone, R₂ est de l'hydrogène ou un groupe acyle R₁, n = 2 ou 3 et x est un nombre entier de 1 à 4,
B) de 1 à 6 % en poids d'un ou plusieurs monoéthanolamides d'acides gras ayant de 12 à 22 atomes de carbone,
C) de 1 à 5 % d'un ou plusieurs produits d'addition non ionogènes d'oxyde d'éthylène sur des alcools gras, des acides gras et des amides d'acide gras ou des alcanolamides d'acides gras, dans lesquels le reste alcoyle gras ou acyle gras porte de 12 à 22 atomes de carbone, sur des alcoylphénols ayant de 8 à 16 atomes de carbone dans le groupe alcoyle et sur des esters partiels d'acide gras et de polyol, choisis dans le groupe formé des monoesters de glycérol, des monoesters de pentaérythrite, ainsi que des mono- et diesters de sorbitanne et d'acide gras ayant de 12 à 22 atomes de carbone avec une valeur de HLB de 12 à 16.

2. Concentrat nacré selon la revendication 1, caractérisé en ce qu'il contient
(A1) de 5 à 8 % en poids d'un mélange de mono- et de distéarate d'éthylèneglycol et
(A2) de 2 à 5 % en poids de distéarate de triéthylèneglycol.

3. Concentrat nacré s'écoulant librement selon les revendications 1 ou 2, caractérisé en ce qu'il contient :
(C1) de 1 à 3 % en poids d'un produit d'addition de 8 à 15 mol d'oxyde d'éthylène sur une coupe d'alcool gras de coco en $C_{12}$-$C_{18}$ et,
(C2) de 0,5 à 2 % en poids d'un produit d'addition de 10 à 20 mol d'oxyde d'éthylène sur une coupe d'alcool gras en $C_{16}$-$C_{18}$ saturée.

4. Concentrat nacré selon les revendications 1 à 3, caractérisé en ce que les composants A et B ensemble représentent 8 à 14 % en poids du concentrat.

5. Concentrat nacré selon les revendications 1 à 4, caractérisé en ce qu'il contient:
(A1) environ 6 % en poids d'un mélange de mono- et de distéarate d'éthylèneglycol dans un rapport pondéral allant de 1:2 à 1:5,
(A2) environ 4 % en poids de distéarate de triéthylèneglycol,
(B) 2 à 4 % en poids d'un monoéthanolamide d'acide gras de coco en $C_{12}$-$C_{18}$
(C1) environ 2 % en poids d'un produit d'addition de 8 à 12 mol d'oxyde d'éthylène sur une coupe d'alcool gras de coco en $C_{12}$-$C_{18}$,

(C2) environ 1 % en poids d'un produit d'addition de 10 à 16 mol d'oxyde d'éthylène sur une coupe d'alcool gras en $C_{16}$-$C_{18}$ saturée.